# EUROPEAN PATENT SPECIFICATION

(11) **EP 2 583 571 B1**
(45) Date of publication and mention of the grant of the patent: **25.06.2014**
(21) Application number: 12188979.4
(22) Date of filing: 18.10.2012
(51) Int. Cl.: A41D 1/08, A41D 17/02, A41D 13/05, A41D 13/04, A61F 5/02

(54) **Improvements relating to chaps**
Verbesserungen im Zusammenhang mit Beinschützern
Améliorations associées aux jambières

(30) Priority: 21.10.2011 GB 201118171; 30.01.2012 GB 201201497; 26.07.2012 GB 201213276
(43) Date of publication of application: 24.04.2013
(73) Proprietor: Elliott, Daniel, Whitby Yorkshire YO21 2PJ (GB)
(72) Inventor: Elliott, Daniel, Whitby Yorkshire YO21 2PJ (GB)
(74) Representative: Marsh, Robin Geoffrey

(56) References cited:
- EP-A1- 0 510 964
- FR-A1- 2 949 298
- US-A- 3 526 221
- US-A- 4 506 391
- US-A- 5 241 704
- US-A- 5 318 507
- US-A- 6 108 819
- US-A1- 2002 020 000
- US-B1- 6 336 908

## Description

This invention relates to chaps and it relates especially, though not exclusively, to protective chaps intended to be worn by farriers and those conducting similar work. Farriers' chaps are sometimes referred to as aprons, and the term "chaps" as used herein is intended to encompass such aprons and like items of protective clothing.

It is usual for farriers to wear chaps, which commonly are made entirely of leather, or at least include significant parts made of leather, to protect their legs and their clothing whilst, for example, supporting or restraining a limb of a horse or other animal on which they are working. Such apparel is well known and is effective to a point.

The work of a farrier, however, dictates that long periods are often spent in uncomfortable positions and this invention aims to provide improved support and/or comfort, particularly for the lumbar or sacroiliac region of the wearer whilst engaged in such work.

Farrier's chaps according to the prior art are disclosed in the document FR. 2.949.298.

Accordingly, the invention provides chaps having an integral belt region extending around the front of a wearer; first and second extension pieces disposed respectively to either end of the belt region and integrally formed with or permanently secured to the chaps; the chaps further comprising a linking belt portion and means for attaching the linking belt portion to each of said extension pieces; said linking belt portion being formed with an enlargement providing a lumbar support and/or a posture aid for the wearer. It will be appreciated that the linking belt portion is needed to complete the rear loop of the belt and that the chaps cannot be worn without the linking portion in place unless the wearer obtains a replacement linking portion (with or without an enlargement) with appropriate fitments for attachment to the extension pieces.

The extension pieces are preferably stitched strongly and permanently into place on the waist-band area of the chaps, and are made of reinforced nylon material.

Further preferably, the attachment of the linking belt portion to the extension pieces is accomplished by way of fitments which allow for quick release/engagement and/or adjustment of the belt as a whole.

Typically, one of the fitments comprises a heavy-duty, quick-release plastics press-fitment, with male and female components thereof being carried by the extension piece and the linking belt portion respectively.

Conveniently, the other fitment affords adjustment of the belt as a whole and comprises a metallic loop, to which the linking portion of the belt is permanently attached; the loop also having a plastics centre-piece that is movable within the loop in known manner, and around which one end of the extension piece is wrapped, tightened and then bent back to overlay the waist area of the chaps. The overlaying portions of the extension piece and the waist band are preferably provided with strong multi micro hook and eye fasteners.

The degree of adjustment that is provided by manipulation of the extension piece relative to the metallic loop, and the robustness of the adjuster are intended to enable the wearer to tighten the composite belt sufficiently for comfortable wear and retention of the chaps under strenuous working conditions.

In some embodiments of the invention, the enlargement comprises an area of the linking belt portion that is widened to create an enlarged support pad of suitable shape. In further embodiments, at least one further pad of the same or a different material is overlain upon and stitched or otherwise secured to the first-mentioned pad, in order to thicken the pad and provide further stiffening and support.

In embodiments with overlain pads, stuffing of a firm medium may be inserted between adjacent pads prior to their being secured together, and entrapped therein by such securement.

In other embodiments, the region between adjacent pads may be left open for access from at least one side, to create a pocket or pouch into which stiffening material may be removably inserted. Any such pocket or pouch may simply be left open, or it may be closed with a flap and a releasable closure device such as a zip or a button arrangement.

In other embodiments of the invention, particularly where the linking portion is made of webbing material, the enlargement providing the lumbar support and/or posture aid is preferably permanently attached to the webbing and comprises an enlarged pad of suitable shape; the pad being attached to and/or enwrapped by a synthetic rubberised material with stuffing of a firm medium enclosed between the pad and the synthetic rubberised material.

In any event, the enlargement may be reinforced or protected within one or more wrapping layers.

In order that the invention may be clearly understood and readily carried into effect, embodiments thereof will now be described, by way of example only, with reference to the accompanying drawings, of which:

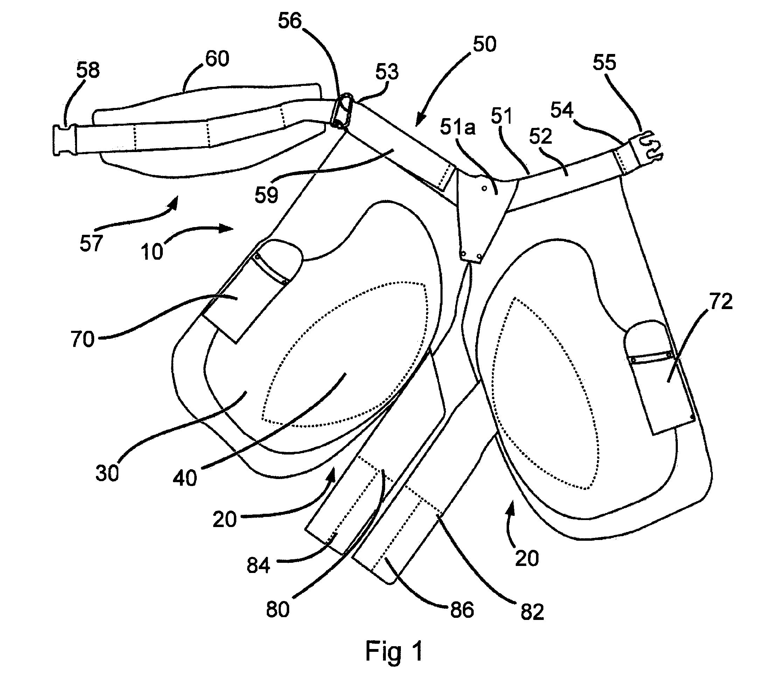

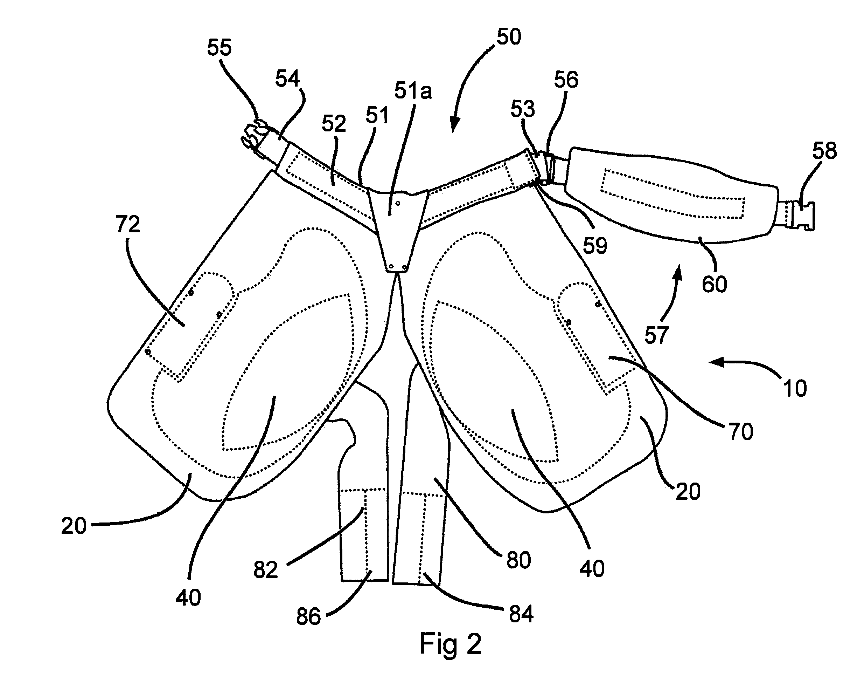

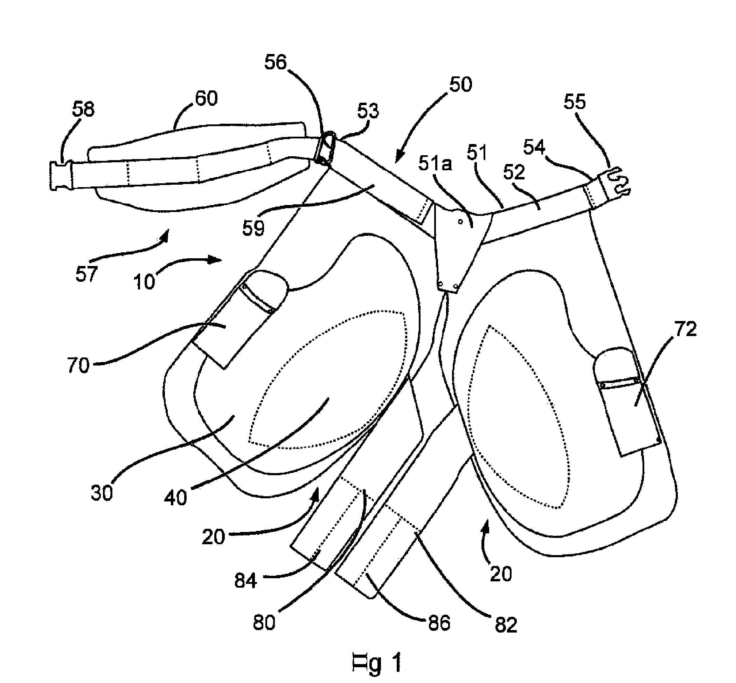

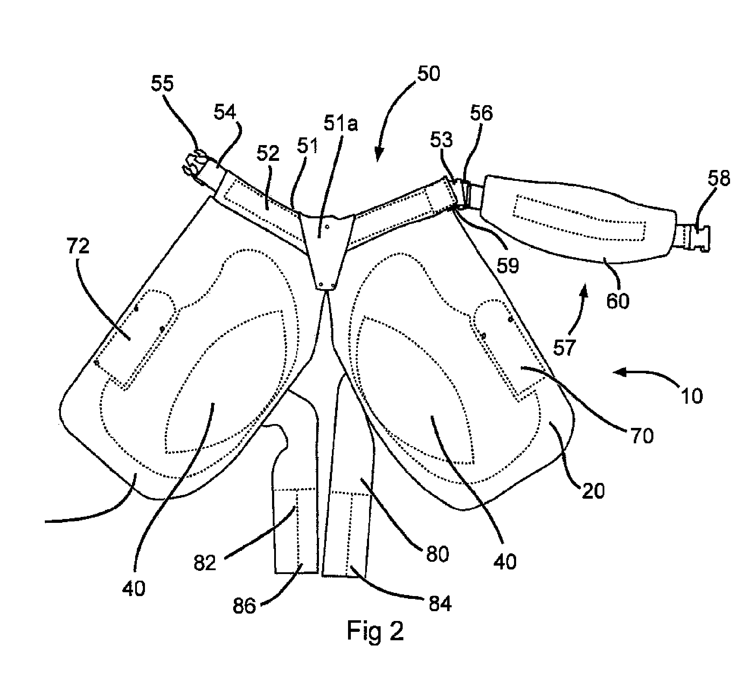

Figures 1 and 2 show, in front and rear elevation respectively, chaps in accordance with an embodiment of the invention.

Referring now to the drawings, the chaps in accordance with one example of the invention are shown at 10. It will be understood that the basic shape and/or construction of the leg-covering parts of the chaps 10 is not material to the invention, and can take any of a number of well-known forms, and the chaps can be configured as riding chaps, for example, in which case the chaps may be additionally (or even primarily) designed to be somewhat decorative, or at least to have a fashionable element.

In this example, however, the chaps are configured as a farrier's apron, and the leg-covering parts 20 are formed primarily of rot-proof and water-proof canvas, with a local covering 30 of chrome leather in areas that are subjected to most wear. In the present example, selected regions, such as 40, of the leather-covered areas 30 further contain an inner padding layer of hard, resilient material, preferably of nitrile material.

A belt, generally shown at 50, is provided to securely hold the chaps in place whilst the wearer performs strenuous tasks, some of which create powerful forces tending to displace the chaps. In this example of the invention, the belt 50 comprises a front portion 51 that is integral with the chaps 10 and comprises a hard resilient material, such as a nitrile material, within a doubled canvas band, with webbing 52 securely attached by stitching to the canvas, thereby forming an abdominal sling of considerable strength.

It is preferred that, as shown in the drawings, the front portion 51, 52 of the belt 50 follows an inverted arcuate form that creates a comfortable shape to the chaps 10 as worn.

The belt 50 further comprises first and second extension pieces 53 and 54, formed as continuations of the webbing 52 and extending respectively from either end of the front belt portion 51. The extension pieces 53 and 54 are effectively integral with the chaps and they are each configured for attachment to respective ends of a further, linking belt portion 57 which comprises the rear loop of the belt 50, i.e. that portion of the belt 50 that is intended to pass around the wearer's back.

The linking belt portion 57 comprises webbing material of the same kind and dimensions as the webbing 52, and the ends of the belt portion 57 are provided with fitments configured for attachment to the extension pieces 53 and 54, so that the belt 50 fits strongly and adjustably to the chaps 10. Importantly, the linking belt portion 57 is formed with a padded enlargement 60 which is permanently stitched to the webbing and provides a lumbar support and/or a posture aid for a wearer of the chaps 10. It will be appreciated that the linking belt portion 57 is needed to complete the belt 50, and that the chaps 10 cannot be worn without the linking portion 57 in place unless the wearer obtains a replacement linking portion (with or without an enlargement) appropriately configured for attachment to the extension pieces.

In this example, the extension piece 54 is securely attached to a fitment 55 which comprises the male element of a multi-pronged, slidably locating quick-release connector; the corresponding female element 58 of which is permanently secured to one end of the linking belt portion 57.

The other end of the linking belt portion 57 is in this example permanently attached to a fitment 56 of known kind, comprising a metallic loop with a serrated plastics cross-member around which the free end 59 of the extension piece 53 is turned and looped back on itself so as to partly overlie the webbing 52 of the front belt portion 51. For added security, it is preferred that the overlying parts of the webbing 52 and 59 are formed with co-operative fields of multi-micro hook and eye material (such as that known by the trade mark Velcro) so that, once the belt 50 has been adjusted to fit by the wearer, the entire belt assembly, and hence the chaps 10, are firmly and reliably held in place.

It will be appreciated that, if preferred, both ends of the belt portion 57 could be fitted with quick-release connectors such as 55, 58 or both ends could be secured to the extension pieces 53 and 54, extending from either end of the front belt portion 51 by means of loop fitments such as 56. Alternatively, the fitments 55/58 and 56 could be used at opposite ends of the belt portion 57 to those shown in the drawings. Indeed, connectors differing from either of those shown in the drawings can be used if preferred, since the actual technique by means of which the belt portions 51 and 57 are securely attached to one another is not material to the invention.

In this example, the belt 50 comprises a two-inch (5cm) wide sling of neoprene, canvas and webbing, with contouring where needed to allow a full range of movement. The enlargement 60 fitted to the rear loop (linking portion) 57 of the belt 50, however, is widened to six inches (15cm), creating an enlargement in the form of a pad providing support for the wearer in the lumbar/sacroiliac region and/or to provide a posture aid.

As can be seen from the drawings, the chaps 10 are effectively formed in two halves, secured together by a strong, doubled-over and somewhat triangularly-shaped front panel 51a, located centrally of the front belt portion 51 and strongly stitched and/or riveted to the front and back of both the belt portion 10 and the leg-covering parts 20 of the chaps. This panel 51a may be made of any material with sufficient strength and resilience to cope with the operational stresses on the chaps when worn, but it is preferred that leather or webbing material is used. Having at least the front belt portion 51 formed in two parts and secured together as shown by the panel 51a assists in forming the desirable inverted arcuate form of the belt referred to above.

It will be appreciated that the pad formed by enlargement 60 is a permanent and irremovable part of the belt portion 57. If necessary, or if desired, the enlargement 60 can further incorporate one or more additional layers of material sewn or otherwise permanently secured together. In multi-layered systems, additional supportive or padding material may, if required, be inserted between one or more pairs of adjacent layers. Any such additional material may be permanently secured in place, e.g. by sewing, or may be removable, as from a pocket or pouch formed between adjacent layers. Any pocket or pouch so formed may be open at its upper surfaces, or may be closable, such as by a zip or buttons. The additional material may or may not have a memory capability, such as memory foam.

One or more pouches or pockets, such as 70 and 72, can be provided, in known manner, on the chaps 10 for holding tools such as knives needed for particular tasks to be carried out by the farrier.

The belt 50 may be formed, in the area thereof which will lie in the hip region of a wearer, with arched regions to readily allow hip movement and thus improve comfort.

It will be appreciated that, without departing from the scope of the invention, the leg-covering parts of the chaps can take any known form to suit an intended specific application, and/or to suit individual choice.

In this example (though not necessarily), the respective coverings for the individual legs of the wearer can be secured in place by means of webbing straps such as 80 and 82 which are each intended to pass round behind a respective leg of the wearer and to attach, by means of their free ends 84 and 86 respectively, to convenient locations on the chaps 10. The attachment to the chaps 10 may be made in any convenient manner; for example by buckles and clasps or by means of strategically positioned fields of multi-micro hook and eye material (such as that known by the trade mark Velcro). The webbing straps 80, 82 may be fitted to the inner (as shown) or outer edges of the leg-covering parts 20 of the chaps 10, and the fitments provided accordingly.

## Claims

1. Chaps (10) having an integral belt region (51) extending around the front of a wearer; first and second extension pieces (53, 54) disposed respectively to either end of the belt region and integrally formed with or permanently secured to the chaps (10); **characterised by** the chaps further comprising a linking belt portion (57) and means (55, 58 and 56) for attaching the linking belt portion (57) to each of said extension pieces (53, 54); said linking belt portion (57) being formed with an enlargement (60) providing a lumbar support and/or a posture aid for the wearer and the integral belt region (51) being configured to form an abdominal sling.

2. Chaps according to claim 1, wherein said extension pieces (53, 54) are stitched strongly and permanently into place on the waist-band area (51) of the chaps (10), and are made of reinforced nylon material.

3. Chaps according to claim 1 or claim 2, wherein the means of attachment of the linking belt portion (57) to at least one of the extension pieces (53, 54) comprises fitments (55, 58) configured for quick release/engagement.

4. Chaps according to claim 3, wherein said fitments (55, 58) comprise a heavy-duty, push-fit plastics connector, with male (55) and female (58) components thereof carried by the extension piece (54) and the linking belt portion (57) respectively.

5. Chaps according to any preceding claim, wherein the means of attachment of the linking belt portion (57) to at least one of the extension pieces (53, 54) comprises adjustable means (56), to enable the wearer to tighten the composite belt (51, 57) sufficiently for comfortable wear and retention of the chaps (10).

6. Chaps according to claim 5, wherein said adjustable means (56) comprises a metallic loop, to which the linking portion (57) of the belt (50) is permanently attached; the loop also having a plastics centre-piece that is movable within the loop and around which one end (59) of the extension piece (53) is wrapped, tightened and then bent back to overlay the waist area of the chaps (10).

7. Chaps according to claim 6, wherein the overlaying portions of the extension piece (53) and the waist band are provided with strong multi micro hook and eye fasteners for mutual engagement and retention.

8. Chaps according to any preceding claim, wherein the enlargement (60) comprises an area of the linking belt portion (57) that is widened to create an enlarged support pad of suitable shape.

9. Chaps according to claim 8, wherein at least one further pad of the same or a different material is overlain upon and stitched or otherwise secured to the first-mentioned pad, in order to thicken the pad and provide further stiffening and support.

10. Chaps according to claim 9, further comprising stuffing of a firm medium inserted between adjacent pads prior to their being secured together, and entrapped therein by such securement.

11. Chaps according to claim 10, wherein said stuffing is permanently secured between said adjacent pads.

12. Chaps according to claim 10, wherein adjacent pads are formed as a pouch or pocket into which said stuffing is removably inserted.

13. Chaps according to any preceding claim, wherein the enlargement (60) is reinforced or protected within one or more wrapping layers.

14. Chaps according to any preceding claim, wherein the integral belt region (51) includes a hard resilient material, such as a nitrile material.

15. Chaps according to claim 14, wherein the hard resilient material of said integral belt region (51) is provided within a doubled canvas band, with webbing securely attached by stitching to the canvas.

## Patentansprüche

1. Sommerschürze (10) für die Hufbearbeitung, mit einem integrierten, um die Vorderseite des Trägers verlaufenden Gurtbereich (51), und mit ersten und zweiten Verlängerungsstücken (53, 54), die an den beiden Enden des Gurtteils angeordnet und integrierend mit der Sommerschürze (10) ausgeführt oder dauerhaft an dieser befestigt sind, **dadurch gekennzeichnet, dass** die Sommerschürze des Weiteren ein Verbindungsgurtteil (57) aufweist, sowie Mittel (55, 58 und 56) zur Befestigung des Verbindungsgurtteils (57) an den besagten Verlängerungsstücken (53, 54), wobei das besagte Verbindungsgurtteil (57) mit einer Vergrößerung (60) ausgeführt ist, die als Lendenwirbel- und/oder Rückenstütze für den Träger fungiert, und dass der integrierte Gurtbereich (51) als Unterleibsgurt ausgeführt ist.

2. Sommerschürze für die Hufbearbeitung gemäß Anspruch 1, bei der die besagten Verlängerungsstücke (53, 54) fest und dauerhaft mit dem Hüftgurtbereich (51) der Sommerschürze (10) vernäht und aus verstärktem Nylonstoff hergestellt sind.

3. Sommerschürze für die Hufbearbeitung gemäß Anspruch 1 oder Anspruch 2, bei der die Mittel zur Befestigung des Verbindungsgurtteils (57) an wenigstens einem der Verlängerungsstücke (53, 54) Vorrichtungen (55, 58) aufweisen, die für ein schnelles Verriegeln bzw. Entriegeln konfiguriert sind.

4. Sommerschürze für die Hufbearbeitung gemäß Anspruch 3, bei der die besagten Vorrichtungen (55, 58) einen hoch beanspruchbaren Kunststoff-Steckverbinder mit Verbindungsstecker (55) bzw. Verbindungsmuffe (57) aufweisen.

5. Sommerschürze für die Hufbearbeitung gemäß einem beliebigen der vorhergehenden Ansprüche, bei der die Mittel zur Befestigung des Verbindungsgurtteils (57) an wenigstens einem der Verlängerungsstücke (53, 54) eine verstellbares Vorrichtung (56) aufweisen, die es dem Träger erlaubt, den zusammengesetzten Gurt (51, 57) für einen festen und bequemen Sitz der Sommerschürze (10) festzuziehen.

6. Sommerschürze für die Hufbearbeitung gemäß Anspruch 5, bei der die verstellbare Vorrichtung (56) eine Metallschlaufe einbezieht, an der das Verbindungsgurtteil (57) des Gurts (50) dauerhaft befestigt ist, wobei die Metallschlaufe ein Kunststoff-Mittelstück aufweist, das innerhalb der Schlaufe beweglich ist, und um ein Ende (59) des Verlängerungsstücks (53) geschlungen, festgezogen und danach nach hinten zurückgebogen wird, so dass es auf dem Hüftteil der Sommerschürze (10) zu liegen kommt.

7. Sommerschürze für die Hufbearbeitung gemäß Anspruch 6, bei dem die aufliegenden Teile des Verlängerungsstücks (53) und der Hüftgurt mit hoch beanspruchbaren, ineinander eingreifenden Mikrohaken- und -ösenelementen zur Befestigung aneinander ausgestattet sind.

8. Sommerschürze für die Hufbearbeitung gemäß einem beliebigen der vorhergehenden Ansprüche, bei dem der vergrößerte Teil (60) einen Bereich des Verbindungsgurtteils (57) einbezieht, der verbreitert ist, so dass er ein vergrößertes Stützpolster von geeigneter Form bildet.

9. Sommerschürze für die Hufbearbeitung gemäß Anspruch 8, bei der wenigstens ein weiteres Polster aus dem gleichen oder einem anderen Material auf dem erstgenannten Polster aufliegend zum Verdicken des Polsters und zu seiner weiteren Versteifung und Abstützung durch Nähen oder auf andere Art und Weise an diesem befestigt wird.

10. Sommerschürze für die Hufbearbeitung gemäß Anspruch 9, des Weiteren umfassend eine Füllung aus festem Medium, die vor Befestigung der einander zugewandten Polster aneinander zwischen diese gelegt und durch Befestigung in dieser Lage gesichert wird.

11. Sommerschürze für die Hufbearbeitung gemäß Anspruch 10, bei der die besagte Füllung dauerhaft zwischen den besagten, einander zugewandten Polstern gesichert ist.

12. Sommerschürze für die Hufbearbeitung gemäß Anspruch 10, bei der die einander zugewandten Polster als Tasche oder Beutel ausgeführt sind, in die bzw. den die besagte Füllung so eingelegt wird, dass sie wieder entfernt werden kann.

13. Sommerschürze für die Hufbearbeitung gemäß einem beliebigen der vorhergehenden Ansprüche, bei der der vergrößerte Teil (60) verstärkt ist, oder eine Lage bzw. mehrere Lagen Schutzumwicklung aufweist.

14. Sommerschürze für die Hufbearbeitung gemäß einem beliebigen der vorhergehenden Ansprüche, bei dem der integrierte Gurtbereich (51) ein hartes, federelastisches Material, beispielsweise einen Nitrilstoff einbezieht.

15. Sommerschürze für die Hufbearbeitung gemäß Anspruch 14, bei dem das harte, federelastische Material des besagten integrierten Gurtbereichs (51) mit einem doppellagigen Leinenband ausgestattet ist, wobei das Gurtband durch Vernähen fest mit dem Leinen verbunden wird.

## Revendications

1. Jambières (10) ayant une région de ceinture intégrale (51) s'étendant autour de la partie avant d'un utilisateur ; des première et seconde pièces d'extension (53, 54) disposées respectivement au niveau de l'une et l'autre extrémité de la région de ceinture et formées d'une seule pièce avec les, ou assujetties de manière permanente aux, jambières (10) ; **caractérisées par** les jambières comportant par ailleurs une partie de ceinture de liaison (57) et des moyens (55, 58 et 56) permettant d'attacher la partie de ceinture de liaison (57) à chacune desdites pièces d'extension (53, 54) ; ladite partie de ceinture de liaison (57) étant formée avec une partie agrandie (60) fournissant un support lombaire et/ou une aide de posture pour l'utilisateur et la région de ceinture intégrale (51) étant configurée pour former une sangle abdominale.

2. Jambières selon la revendication 1, dans lesquelles lesdites pièces d'extension (53, 54) sont cousues en place de manière résistante et permanente sur la région de ceinture (51) des jambières (10), et sont faites à partir d'un matériau de nylon renforcé.

3. Jambières selon la revendication 1 ou la revendication 2, dans lesquelles le moyen de fixation de la partie de ceinture de liaison (57) sur au moins l'une des pièces d'extension (53, 54) comporte des dispositifs de mise en place (55, 58) configurés à des fins de fermeture/ouverture rapide.

4. Jambières selon la revendication 3, dans lesquelles lesdits dispositifs de mise en place (55, 58) comportent un connecteur plastique de type à ajustement par pression d'utilisation intensive, les composants mâle (55) et femelle (58) de celui-ci étant portés par la pièce d'extension (54) et la partie de ceinture de liaison (57) respectivement.

5. Jambières selon l'une quelconque des revendications précédentes, dans lesquelles le moyen de fixation de la partie de ceinture de liaison (57) sur au moins l'une des pièces d'extension (53, 54) comporte un moyen ajustable (56), pour permettre à l'utilisateur de serrer suffisamment la ceinture composite (51, 57) pour un port confortable et une retenue des jambières (10).

6. Jambières selon la revendication 5, dans lesquelles ledit moyen ajustable (56) comporte une boucle métallique, à laquelle la partie de liaison (57) de la ceinture (50) est attachée de manière permanente ; la boucle ayant aussi une pièce centrale plastique qui est mobile à l'intérieur de la boucle et autour de laquelle une extrémité (59) de la pièce d'extension (53) est enveloppée, serrée puis repliée à des fins de couverture de la zone au niveau de la taille des jambières (10).

7. Jambières selon la revendication 6, dans lesquelles les parties de couverture de la pièce d'extension (53) et de la ceinture comportent des attaches à multiples micro agrafes et portes à des fins de mise en prise et de retenue, et ce mutuellement.

8. Jambières selon l'une quelconque des revendications précédentes, dans lesquelles la partie agrandie (60) comporte une zone de la partie de ceinture de liaison (57) qui est élargie pour créer un coussinet de support agrandi de forme appropriée.

9. Jambières selon la revendication 8, dans lesquelles au moins un autre coussinet du même matériau ou d'un matériau différent est reposé sur et cousu ou autrement assujetti sur le premier coussinet mentionné, à des fins d'épaississement du coussinet et de mise en oeuvre de plus de renfort et de support.

10. Jambières selon la revendication 9, comportant par ailleurs le garnissage d'un milieu ferme inséré entre des coussinets adjacents avant leur assujettissement ensemble, et pris au piège dans ceux-ci par un tel assujettissement.

11. Jambières selon la revendication 10, dans lesquelles le garnissage est assujetti de manière permanente entre lesdits coussinets adjacents.

12. Jambières selon la revendication 10, dans lesquelles des coussinets adjacents sont formés comme une pochette ou poche dans laquelle ledit garnissage est inséré de manière amovible.

13. Jambières selon l'une quelconque des revendications précédentes, dans lesquelles la partie agrandie (60) est renforcée ou protégée à l'intérieur d'une ou de plusieurs couches à envelopper.

14. Jambières selon l'une quelconque des revendications précédentes, dans lesquelles la région de ceinture intégrale (51) comprend un matériau élastique dur, tel un matériau de nitrile.

15. Jambières selon la revendication 14, dans lesquelles le matériau élastique dur de ladite région de ceinture intégrale (51) est mis en oeuvre à l'intérieur d'une bande de canevas doublée, avec une sangle attachée de manière sûre par des points de couture sur le canevas.
